# EUROPEAN PATENT SPECIFICATION

(11) **EP 4 015 505 B1**
(45) Date of publication and mention of the grant of the patent: **05.08.2026**
(21) Application number: 21180903.3
(22) Date of filing: 22.06.2021
(51) Int. Cl.: C07D 215/28

(54) **IMPROVED PROCESS FOR PRODUCING NITROXOLINE**
VERBESSERTES VERFAHREN ZUR HERSTELLUNG VON NITROXOLIN
PROCÉDÉ AMÉLIORÉ POUR LA PRODUCTION DE NITROXOLINE

(30) Priority: 15.12.2020 IN 202021054433
(43) Date of publication of application: 22.06.2022
(73) Proprietor: Kopran Research Laboratories Limited, Mumbai 400 018, Maharashtra (IN)
(72) Inventor: PANDEY, Anand Kumar, 230128 Dist Pratapgarh, Uttar Pradesh (IN); DONGARGE, Mahesh Yogiraj, 431603 Nanded, Maharashtra (IN); CHAUGULE, Suryabhan Baban, 414305 Dist. Ahmednagar, Maharashtra (IN)
(74) Representative: Isarpatent

(56) References cited:
- ISAEV A A ET AL: "Technology of Preparing 8-Hydroxy-5-nitroquinoline", CHEMISTRY OF HETEROCYCLIC COMPOUNDS, KLUWER ACADEMIC PUBLISHERS-PLENUM PUBLISHERS, NL, vol. 41, no. 8, 1 August 2005 (2005-08-01), pages 1027 - 1030, XP019276417, ISSN: 1573-8353

## Description

### FIELD OF INVENTION

The present invention relates to an improved and cost effective process for preparation of Nitroxoline (5-nitro-8-oxyquinoline) free from process related genotoxic impurities. The process of the present invention significantly controls the formation of the undesired side-product i.e. 7-Nitro nitroxoline during oxidation of 5-nitroso-8-oxyquinoline and results in improved yield and purity of the product.

### BACKGROUND OF THE INVENTION

Nitroxoline i.e. 5-nitro-8-hydroxyquinoline with antibacterial activity is conventionally prepared by nitrosating 8-oxyquinoline with sodium nitrite, followed by oxidizing the resulting 5-nitroso-8-hydroxyquinoline with nitric acid. In the conventional oxidation process there is substantial formation of the undesired impurity 7-Nitro nitroxoline which is very difficult to remove and requires extensive purification stages to obtain pure nitroxoline. The purification steps are tedious and reduce the yield significantly.

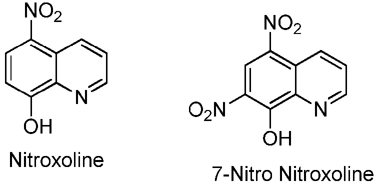

RU2702004C2 relates to a process of producing pharmacopoeial 5-nitro-8-hydroxyquinoline using technical nitroxoline. The process includes adding in the first load acetone, activated carbon to technical nitroxoline followed by adding commodity acetone in the second load. The ratio of the commodity acetone to the regenerated acetone of the first load is 2:3. This is followed by heating the reaction mass to a temperature of 60 ± 5°C for 1.0-2.0 hours. The reaction mass is then transferred through a filter in the crystallizer reactor into which purified water is pre-loaded, after which the reaction mass is cooled to a temperature of 20 ± 5° C and held for 1.5-3.0 hours. The resulting pharmacopoeial nitroxoline paste is filtered, squeezed and washed with purified water, then the content of chlorides and sulfates in the paste are analyzed, until the content of chlorides in it is not more than 0.02% and sulfates not more than 0.02%, after that the pharmacopoeial nitroxoline paste is transferred to drying, which is carried out at a temperature of 60±15°C until a mass loss upon drying of not more than 0.5% is achieved.

SU609284A1 discloses a process for preparation of pharmacopoeial nitroxoline, wherein the oxidation of 5-nitroso-8-hydroxyquinoline is carried under controlled conditions in organic solvent i.e. acetic acid, and purified in acetone at a temperature of 20-50° C. The purification of the resulting product is carried out by transferring it to the hydrochloric acid and crystallizing it from dilute hydrochloric acid. The process is not advantageous as it cannot control the formation of 7-nitro nitroxoline, which is a process related genotoxic impurity which does form during oxidation of 5-nitroso-8-hydroxyquinoline. The intermediate stage 5-nitroso-8-hydroxyquinoline is also considered a genotoxic impurity, these impurities do not get removed even after multiple purification as they have similar solubility profile. The nitroxoline and 5-nitro nitroxoline are poorly soluble in most of the organic solvents, it requires about 40-50 volume acetone for single purification stage and it needs multiple purifications to meet the desired quality. But the multiple purifications stages do not offer consistent result to meet the acceptance criteria of genotoxic impurity limits.

A. A. Isaev et al., Chemistry of Heterocyclic Compounds (2005), 41, 1027 describe a two-stage method for the preparation of 8-hydroxy-5-nitroquinoline.

All the reported processes of nitroxoline are less productive, cumbersome and not suitable for commercial manufacturing for ICH grade API which does comply with stringent genotoxic impurities limits. The present inventors found that nitroxoline obtained by the conventional methods is high in process related genotoxic impurities (7-nitro nitroxoline and 5-nitroso-8-hydroxyquinoline), need multiple purification stages, poor in description, greenish brown to soil color powder without gloss which is unpleasant, has poor flow property, not desired by the formulators for tableting.

Therefore, there is a need in the art to provide an improved process for preparation of ICH grade nitroxoline which is cost effective and practical for commercial manufacturing. The present inventors surprisingly found that ICH grade nitroxoline, as yellow to yellowish brown shining powder free from process related genotoxic impurities can be obtained using a unique solvent system both during the oxidation step and during re-crystallization. This remains the objective of the invention.

### SUMMARY OF THE INVENTION

In accordance with the above, the present invention provides a process for producing ICH grade nitroxoline, wherein said process comprising;
i. Oxidizing 5-Nitroso-8-hydroxy quinoline with an oxidizing agent in water and water miscible organic solvent mixture at a temperature in the range of 10-35°C to obtain crude nitroxoline; and
ii. Recrystallizing the crude nitroxoline of step (i) from acetone, water, DMF or mixture thereof to obtain nitroxoline as yellow to yellowish brown shining powder free from process related genotoxic impurities such as 7-Nitro nitroxoline, 5-Nitroso-8-hydroxyquinoline and 5,7-Dinitro-8-hydroxyquinoline.

The water miscible organic solvent during oxidation in step (i) is selected from methanol, ethanol, propanol, dimethyl formamide (DMF), N,N dimethyl acetamide (DMAc), dimethyl sulfoxide (DMSO) or mixtures thereof.

The oxidizing reagent is nitric acid.

The recrystallization of crude nitroxoline in step (ii) is carried out using DMF, acetone, water or mixtures thereof to obtain pure nitroxoline as yellow to yellowish brown powder containing less than 0.005% of undesired 7-Nitro Nitroxoline impurity and 5-Nitroso-8-hydroxyquinoline, which is below quantification limit (BQL).

In another aspect, the 5-Nitroso-8-hydroxy quinoline is obtained by nitration of 8-hydroxyquinoline by a process known in the art.

### DETAILED DESCRIPTION OF THE INVENTION

The present invention will now be explained in detail with reference to its various preferred as well as optional embodiment, which, however should not be construed to limit the scope of the invention.

In an embodiment, the present invention relates to an improved, cost effective process for producing ICH grade nitroxoline wherein said process comprising;
i. Oxidizing 5-Nitroso-8-hydroxy quinoline with an oxidizing agent in mixture of water and water miscible organic solvent at a temperature in the range of 10-35°C to obtain crude nitroxoline; 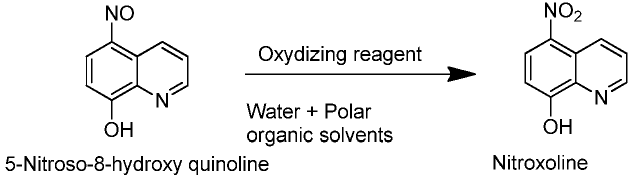 and
ii. Recrystallizing the crude nitroxoline of step (i) from acetone, water, DMF or mixtures thereof to obtain nitroxoline as yellow to yellowish brown shining powder free from process related genotoxic impurities such as 7-Nitro nitroxoline, 5-Nitroso-8-hydroxyquinoline and 5,7-Dinitro-8-hydroxyquinoline.

According to the present process step (i) water and polar organic solvent was charged to 5-Nitroso-8-hydroxy quinoline in a reactor and the mixture was stirred and cooled to about 10°C. To the cooled mixture was added oxidizing agent at a temperature in the range of 10-35°C over a period of about 3-5hrs. After completion of the reaction the pH was adjusted with a base, the slurry was filtered, washed and dried to yield crude nitroxoline as greenish powder.

The water miscible organic solvent is selected from methanol, ethanol, propanol, dimethyl formamide (DMF), N,N dimethyl acetamide (DMAc), dimethyl sulfoxide (DMSO) or mixtures thereof; preferably the organic solvent is lower alcohol.

The oxidizing reagent for step (i) is nitric acid.

The re-crystallization is carried out at a temperature in the range of 20-100°C.

The process step (ii) comprises adding dimethyl formamide (DMF) to crude nitroxoline and raising the temperature. Stirring the mixture followed by adding water slowly and cooling the reaction mixture to room temperature, filtering. Checked the 7-Nitro nitroxoline content in the wet material and the process was repeated until the 7-nitro nitroxoline content is NMT 0.5%. Dried the wet material to obtain Nitroxoline as brownish green color powder.

This was followed by re-crystallization of the above obtained purified nitroxoline which comprises heating the purified nitroxoline with DMF to obtain clear solution. Activated carbon is then added, stirred and filtered to remove the activated carbon residue. Acetone was added slowly to the filtrate to crystallize Nitroxoline. Filtered the slurry, dried the wet material under reduced pressure to get the Nitroxoline as light yellow brown color powder of high purity wherein the genotoxic impurities are below quantification limit (BQL) analyzed with HPLC system and confirm the genotoxic compliance tested with advance LCMS-MS machine.

In another embodiment, the compound 5-Nitroso-8-hydroxy quinoline is prepared by nitration of 8-hydroxyquinoline. Accordingly, to the compound 8-hydroxyquinoline was charged in water at ambient temperature. The mixture was cooled and added conc. sulphuric acid at 0-40°C. Slowly added aqueous solution of sodium nitrite and the mixture was stirred at the same temperature. Adjusted the pH to 5.0 with acetic acid. The slurry was filtered, purified and dried to obtain the desired product.

In another embodiment, the present invention discloses an improved, cost effective process for producing ICH grade nitroxoline wherein said process comprising;
i. Nitrating 8-hydroxyquinoline to obtain 5-Nitroso-8-hydroxy quinoline by a process known in art;
ii. Oxidizing 5-Nitroso-8-hydroxy quinoline of step (i) with an oxidizing agent in water and water miscible organic solvent at a temperature in the range of 10-35°C to obtain crude nitroxoline; 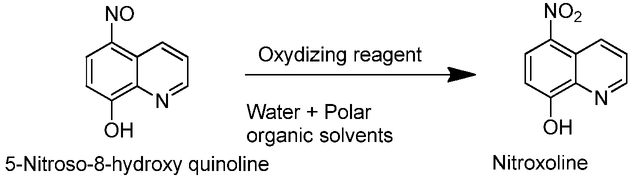 and
iii. Recrystallizing the crude nitroxoline of step (ii) from acetone, water, DMF or mixture thereof to obtain ICH grade nitroxoline as yellow to yellowish brown powder free from process related genotoxic impurities such as 7-Nitro nitroxoline and 5-Nitroso-8-hydroxyquinoline.

The water miscible organic solvent for step (i) is selected from methanol, ethanol, propanol, dimethyl formamide (DMF), N,N dimethyl acetamide (DMAc), dimethyl sulfoxide (DMSO) or mixtures thereof.

The oxidizing reagent for step (i) is nitric acid.

The re-crystallization is carried out at a temperature in the range of 20-100°C.

In an embodiment, pure Nitroxoline prepared by the present process as yellow to yellowish brown powder contain less than 0.01% of undesired 7-nitro nitroxoline impurity and 5-Nitroso-8-hydroxy quinoline which is below quantification Limit (BQL) analyzed HPLC analysis and confirm the genotoxic limits if tested with LCMS-MS system.

In a preferred embodiment, pure Nitroxoline prepared by the present process as yellow to yellowish brown powder contain less than 0.005% of undesired 7-Nitro Nitroxoline impurity and 5-Nitroso-8-hydroxyquinoline which is below quantification Limit (BQL).

The advantage of the present process is that the use of solvent mixture of water and organic solvent during the oxidation of 5-Nitroso-8-hydroxy Quinoline significantly controls the formation of 7-Nitro nitroxoline which is considered as genotoxic material. The genotoxic material is a process related impurity that gets generated during intermediate steps. The use of DMF- acetone mixture or pure acetone improves the quality of nitroxoline evident from its change from brownish green soil color material to yellow to yellowish brown shining material.

The free from process related genotoxic impurities such as 7-Nitro nitroxoline and 5-Nitroso-8-hydroxyquinoline means these impurities are in compliance with genotoxic impurities limit which is less than 12.5ppm and 26ppm respectively.

### Experimental:

### Example 1&2: Preparation of 5-Nitroso-8-hydroxyquinoline

To a 3 neck round bottom flask was charged water (1000 ml) and 8-hydroxyquinoline (100gm) at ambient temperature. It was then cooled and added concentrated sulphuric acid at 15-20°C. Slowly added the aqueous solution of sodium nitrite (20gm, 200ml water). The mixture was stirred for 80min at the same temperature. Adjusted the pH to 5.0 with sodium hydroxide solution. The slurry was filtered and the wet cake was purified with methanol, dried to yield the title product.

| Example | Batch No. | Batch Size (gm) | Unreacted 8-HQ below 1.0% | HPLC Purity |
|---|---|---|---|---|
| 1. | RD/NTX/I/20/098 | 100 | 0.50 | 99.25 |
| 2. | RD/NTX/I/20/099 | 100 | ND | 98.89 |

### Example 3 & 4: Preparation of Nitroxoline crude (present invention)

To a 3 neck round bottom flask was charged water (3vol) and methanol (1vol) at ambient temperature and added 5-Nitroso-8-hydroxyquinoline (50gm) material, stirred and cooled to 10°C. The concentrated nitric acid was slowly added at 10-35°C over a period of 3-5hr. After completion of reaction adjusted pH 4-5 with aqueous sodium hydroxide. The slurry was filtered and the wet cake was washed with water, dried to obtain the title product as greenish powder. Yield: 100gm

| Example | Batch No. | Batch size (gm) | Unreacted 5-nitroso-8-hydroxyquinoline | 7-Nitro Nitroxoline | Purity by HPLC |
|---|---|---|---|---|---|
| 3. | RD/NTX/II/20/100 | 100 | 0.17% | 0.29% | 99.44% |
| 4. | RD/NTX/II/20/101 | 100 | 0.15% | 0.24% | 99.59% |

### Example 5 & 6: Preparation of Nitroxoline technical

To a 3 neck round bottom flask was charged crude Nitroxoline and dimethyl formamide (4vol) at ambient temperature and raised the temperature to 70-80°C. Stirred the mixture and added water (8vol) slowly, cooled to room temperature and filtered. The wet material was dried under hot air oven obtain the title product as brownish green colour powder.

| Example | Batch No. | Batch Size (gm) | HPLC purity | | |
|---|---|---|---|---|---|
| | | | Nitroxoline | 5-Nitroso-8-hydroxyquinoline (NMT 0.05%) | 7-Nitro Nitroxoline (NMT 3.0%) |
| 5. | RD/NTX/III/20/100 | 100 | 99.86 | 0.02 | 0.05 |
| 6. | RD/NTX/III/20/101 | 100 | 99.91 | 0.02 | 0.03 |

The Nitroxoline obtained in example 5& 6 was brownish green colour powder with improper lumpy poor texture the process required an additional crystallization step to improve the powder properties of the material and remove the process related genotoxic impurities.

### Example 7&8: Preparation of Nitroxoline pure

To a 3 neck round bottom flask was charged Nitroxoline technical material obtained from example 5 & 6 separately and DMF (4vol) and raised the temperature to 60-80°Cto get the clear solution. Activated carbon was added, stirred and filtered out the activated carbon residue. Acetone (8 Vol) was added slowly into filtrate to crystallize Nitroxoline. Filtered the slurry, the wet material was dried under reduced pressure to get the title product as light yellow colour powder with HPLC purity 99.9%, and 5-Nitroso-8-hydroxyquinoline and 7-Nitro Nitroxoline are below quantification Limit, (BQL, i.e. 0.005% with HPLC method of analysis. Yield: 70-80%.

| Example | Batch No. | Batch Size (gm) | LCMS-MS Analysis for genotoxic impurities | |
|---|---|---|---|---|
| | | | 5-Nitroso-8-hydroxyquinoline (NMT 26ppm) | 7-Nitro Nitroxoline (NMT 12.5ppm) |
| 7. | RD/NTX/VI/20/100 | 90 | ND | ND |
| 8. | RD/NTX/VI/20/101 | 90 | ND | ND |

### Example 9&10: Preparation of Nitroxoline pure

To a 1kl of glass reactor was charged the Nitroxoline technical material obtained from example 5 & 6 separately and DMF (4vol) and raised the temperature to 60-80°C to get the clear solution. Activated carbon was added, stirred and filtered out the activated carbon residue. Acetone (8 Vol) was added slowly into filtrate to crystallize Nitroxoline. Filtered the slurry, the wet material was dried under reduced pressure to get the title product as light yellow colour powder and confirmed 5-Nitroso-8-hydroxyquinoline and 7-Nitro Nitroxoline are not detected tested with LCMS-MS machine.

| Example. | Batch No. | Batch Size (Kg) | HPLC purity | |
|---|---|---|---|---|
| | | | 5-Nitroso-8-hydroxyquinoline ((NMT 0.05%)) | 7-Nitro Nitroxoline (NMT 0.5%)) |
| 9. | NTX/P2008037 | 40 | ND | ND |
| 10. | NTX/P2008038 | 39 | ND | ND |

**Table 1: Process Comparison**

| **Attributes** | **Prior Art Processes** | **Present Process** |
|---|---|---|
| Oxidation of 5-nitroso-8-hydroxy quinoline | Water | Water and a water miscible organic solvent |
| 7-Nitro Nitroxoline impurity formation during reaction | 2.5 to 10% | Less than 1.0% |
| Number of Purification required to meet the HPLC limit test for 7-Nitro Nitroxoline i.e. 12.5ppm | 2-3 purification cycle is required | Only one purification cycle |
| Yield (w/w; from 8-Hydroxy Quinoline) | 0.40 - 0.50 | 0.70 - 0.85 |

The impurity formation has been substantially controlled with the present invention, improved process of nitroxoline manufacturing which eventually increased the process throughput and much purer product.

## Claims

1. A process for producing ICH grade nitroxoline **characterized in that** said process comprising;
i. Oxidizing 5-Nitroso-8-hydroxy quinoline with nitric acid in mixture of water and water miscible organic solvents at a temperature in the range of 10-35°C to obtain crude nitroxoline; 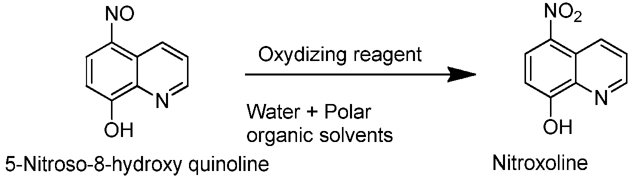 and
ii. Recrystallizing the crude nitroxoline of step (i) from acetone, water, DMF or mixture thereof to obtain ICH grade nitroxoline as yellow to yellowish brown shining powder free from process related genotoxic impurities such as 7-Nitro nitroxoline, 5-Nitroso-8-hydroxyquinoline and 5,7-Dinitro-8-hydroxyquinoline,
wherein the water miscible organic solvent for step (i) is selected from methanol, ethanol, propanol, dimethyl formamide (DMF), N,N dimethyl acetamide (DMAc), dimethyl sulfoxide (DMSO) or mixtures thereof.

2. The process as claimed in claim 1, wherein re-crystallization of step (ii) is carried out at a temperature in the range of 20-100°C.

3. The process for producing ICH grade nitroxoline as claimed in claim 1, comprising;
i. Nitrating 8-hydroxyquinoline to obtain 5-Nitroso-8-hydroxy quinoline by a process known in art;
ii. Oxidizing 5-Nitroso-8-hydroxy quinoline of step (i) with nitric acid in mixture of water and water miscible organic solvent at a temperature in the range of 10-35°C to obtain crude nitroxoline; 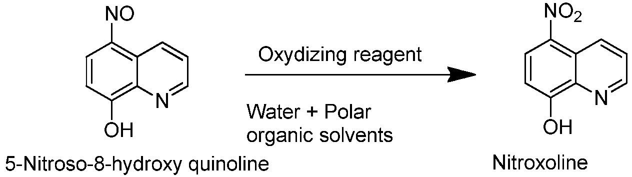 and
iii. Recrystallizing the crude nitroxoline of step (ii) from acetone, water, DMF or mixture thereof to obtain ICH grade nitroxoline as yellow to yellowish brown shining powder free from process related genotoxic impurities such as 7-Nitro nitroxoline, 5-Nitroso-8-hydroxyquinoline
wherein the water miscible organic solvent for step (ii) is selected from methanol, ethanol, propanol, dimethyl formamide (DMF), N,N dimethyl acetamide (DMAc), dimethyl sulfoxide (DMSO) or mixtures thereof.

4. The process as claimed in claim 3, wherein the re-crystallization of step (iii) is carried out at a temperature in the range of 20-100°C.

## Patentansprüche

1. Verfahren zur Herstellung von Nitroxolin in ICH-Qualität, **dadurch gekennzeichnet, dass** das Verfahren umfasst:
i. Oxidieren von 5-Nitroso-8-hydroxychinolin mit Salpetersäure in einer Mischung aus Wasser und wassermischbaren organischen Lösungsmitteln bei einer Temperatur im Bereich von 10-35 °C^{,} um rohes Nitroxolin zu erhalten; 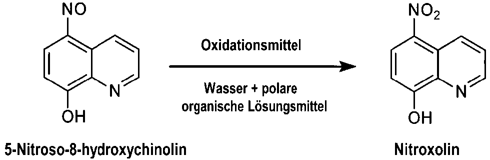 und
ii. Umkristallisieren des rohen Nitroxolins aus Schritt (i) aus Aceton, Wasser, DMF oder einer Mischung davon, um Nitroxolin in ICH-Qualität als gelbes bis gelblichbraunes, glänzendes Pulver zu erhalten, das frei von prozessbedingten genotoxischen Verunreinigungen wie 7-Nitro-nitroxolin, 5-Nitroso-8-hydroxychinolin und 5,7-Dinitro-8-hydroxychinolin ist,
wobei das mit Wasser mischbare organische Lösungsmittel für Schritt (i) ausgewählt ist aus Methanol, Ethanol, Propanol, Dimethylformamid (DMF), N,N-Dimethylacetamid (DMAc), Dimethylsulfoxid (DMSO) oder Mischungen davon.

2. Verfahren nach Anspruch 1, wobei die Umkristallisation von Schritt (ii) bei einer Temperatur im Bereich von 20-100 °C durchgeführt wird.

3. Verfahren zur Herstellung von Nitroxolin in ICH-Qualität nach Anspruch 1, umfassend:
i. Nitrierung von 8-Hydroxychinolin, um 5-Nitroso-8-hydroxychinolin durch ein in der Technik bekanntes Verfahren zu erhalten;
ii. Oxidieren von 5-Nitroso-8-hydroxychinolin aus Schritt (i) mit Salpetersäure in einer Mischung aus Wasser und einem mit Wasser mischbaren organischen Lösungsmittel bei einer Temperatur im Bereich von 10-35 °C^{,} um rohes Nitroxolin zu erhalten; 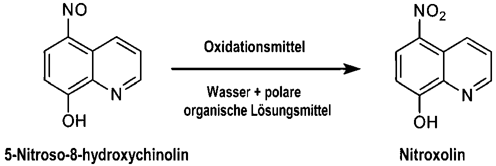 und
iii. Umkristallisieren des rohen Nitroxolins aus Schritt (ii) aus Aceton, Wasser, DMF oder einer Mischung davon, um Nitroxolin in ICH-Qualität als gelbes bis gelblichbraunes, glänzendes Pulver zu erhalten, das frei von prozessbedingten genotoxischen Verunreinigungen wie 7-Nitro-Nitroxolin und 5-Nitroso-8-hydroxychinolin ist
wobei das wassermischbare organische Lösungsmittel für Schritt (ii) aus Methanol, Ethanol, Propanol, Dimethylformamid (DMF), N,N-Dimethylacetamid (DMAc), Dimethylsulfoxid (DMSO) oder Mischungen davon ausgewählt ist.

4. Verfahren nach Anspruch 3, wobei die Umkristallisation von Schritt (iii) bei einer Temperatur im Bereich von^{20 bis}100 °C durchgeführt wird.

## Revendications

1. Procédé de production de nitroxoline de qualité ICH, **caractérisé en ce que** ledit procédé comprend :
i. l'oxydation de la 5-nitroso-8-hydroxyquinoléine avec de l'acide nitrique dans un mélange d'eau et de solvants organiques miscibles à l'eau, à une température comprise entre^{10 et} 35 °C^{,} pour obtenir de la nitroxoline brute ; 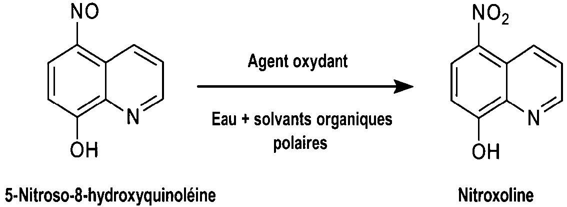 et
ii. la recristallisation de la nitroxoline brute de l'étape (i) à partir d'acétone, d'eau, de DMF ou d'un mélange de ceux-ci pour obtenir de la nitroxoline de qualité ICH sous forme de poudre brillante de couleur jaune à brun jaunâtre, exempte d'impuretés génotoxiques liées au procédé telles que la 7-nitro-nitroxoline, la 5-nitroso-8-hydroxyquinoléine et la 5,7-dinitro-8-hydroxyquinoléine,
dans laquelle le solvant organique miscible à l'eau pour l'étape (i) est choisi parmi le méthanol, l'éthanol, le propanol, le diméthylformamide (DMF), le N,N-diméthylacétamide (DMAc), le diméthylsulfoxyde (DMSO) ou des mélanges de ceux-ci.

2. Procédé selon la revendication 1, dans lequel la recristallisation de l'étape (ii) est effectuée à une température comprise entre^{20 et} 100 °C.

3. Procédé de production de nitroxoline de qualité ICH selon la revendication 1, comprenant :
i. la nitration de la 8-hydroxyquinoléine pour obtenir de la 5-nitroso-8-hydroxyquinoléine par un procédé connu dans l'art ;
ii. l'oxydation de la 5-nitroso-8-hydroxyquinoléine de l'étape (i) avec de l'acide nitrique dans un mélange d'eau et d'un solvant organique miscible à l'eau à une température comprise entre^{10 et} 35 °C pour obtenir de la nitroxoline brute ; 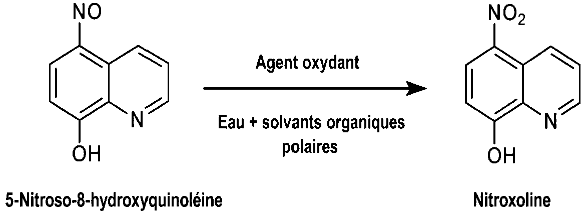 et
iii. Recristalliser la nitroxoline brute de l'étape (ii) à partir d'acétone, d'eau, de DMF ou d'un mélange de ceux-ci pour obtenir de la nitroxoline de qualité ICH sous forme de poudre brillante jaune à brun jaunâtre, exempte d'impuretés génotoxiques liées au procédé telles que la 7-nitro-nitroxoline et la 5-nitroso-8-hydroxyquinoléine
dans lequel le solvant organique miscible à l'eau utilisé pour l'étape (ii) est choisi parmi le méthanol, l'éthanol, le propanol, le diméthylformamide (DMF), le N,N-diméthylacétamide (DMAc), le diméthylsulfoxyde (DMSO) ou des mélanges de ceux-ci.

4. Procédé selon la revendication 3, dans lequel la recristallisation de l'étape (iii) est effectuée à une température comprise entre^{20 et}100 °C.
